# EUROPEAN PATENT APPLICATION

(11) **EP 4 091 664 A1**
(43) Date of publication of application: **23.11.2022**
(21) Application number: 20914514.3
(22) Date of filing: 13.01.2020
(51) Int. Cl.: A61N 1/36, G16H 50/20, G16Y 40/10, G16Y 10/60, A61B 5/053

(54) **PROCESS AND SYSTEM FOR ARTIFICIAL ACTIVATION AND MONITORING OF NEUROMUSCULAR TISSUE BASED ON ARTIFICIAL INTELLIGENCE**

(71) Applicant: Zandona Freitas, Ângelo Eustáquio, 30535-690 Belo Horizonte (BR); Avelar, Bruno Silveira, 30240-410 Belo Horizonte (BR); Santos, Daniel Alexandre Correia, 34007-164 Nova Lima (BR); Alcåntara, Diógenes De Oliveira, CEP 30575-010 (BR); Abrão, Humberto Jonas Fátima, MG CEP 30 320 630 (BR); De Souza E Silva, Paulo Eugênio Oliveira, 71906-500 Brasília (BR); de Almeida, Pedro Villaça, 34006-074 Nova Lima (BR)
(72) Inventor: Zandona Freitas, Ângelo Eustáquio, 30535-690 Belo Horizonte (BR); Avelar, Bruno Silveira, 30240-410 Belo Horizonte (BR); Santos, Daniel Alexandre Correia, 34007-164 Nova Lima (BR); Alcåntara, Diógenes De Oliveira, CEP 30575-010 (BR); Abrão, Humberto Jonas Fátima, MG CEP 30 320 630 (BR); De Souza E Silva, Paulo Eugênio Oliveira, 71906-500 Brasília (BR); de Almeida, Pedro Villaça, 34006-074 Nova Lima (BR)
(74) Representative: Ferreira Magno, Fernando Antonio
(86) International application number: PCT/BR2020/050005
(87) International publication number: WO 2021/142518

(57) **Abstract**

The invention is characterized by a set of innovations in the technique called Artificial Neuromuscular Activation and Monitoring System - SAMNA, based on artificial intelligence. The invention automatically enables accurate diagnosis and effective personalized treatment for each type of muscle group, in different patients, allows continuous monitoring of the neuromuscular condition, treating and preventing vascular, osteotendinous and neuromuscular disorders with artificial and automated neuromuscular activation using cloud data processing and machine learning, allows the diagnosis/treatment of neuromuscular disorders on a continuous basis, locally or remotely. The hardware, firmware and software resources allow the acquisition of neurophysiological parameters and application of therapeutic parameters automatically, eliminating the use of sensors, making use of the electrotherapy electrodes themselves and performing their actions independently, personalized and coordinated, continuously controlling the contraction level.

## Description

### FIELD OF THE INVENTION

The present invention belongs predominantly to the health area, with a focus on physiotherapy applications aimed at activating neuromuscular tissues. In a complementary way, the technology developed is also located in the field of Information and Communication Technologies (ICTs), as it comprises knowledge related to devices that employ artificial intelligence and the internet of things (IoT - internet of things). More particularly, the invention addresses matter pertaining to automatic, non-invasive equipment, aimed at continued neuromuscular electrical activation.

The developed technology consists of a system equipped with an IoT resource, with horizontal monitoring of the neuromuscular condition, processing of data in the cloud, from databases using knowledge of b ig d ata, also employing learning techniques machine, also known as machine learn and technique are to prevent, treat and diagnose disorders of the neuromuscular system by means of neurophysiological tests and artificial neuromuscular activation. All of these actions are carried out automatically, continuously, locally or remotely. For this, the system has hardware, firmware and software resources that allow the control of therapeutic and diagnostic parameters, ensuring the appropriate adjustments for each muscle group.

The invention, through the listed resources, makes it feasible to apply a more effective, customized and personalized dose for each muscle group of each patient. The invention is also capable of executing different therapeutic protocols that are pre-programmed in the system and new protocols can be loaded remotely from a server that can be in a cloud structure and that is an integral part of the system. These same therapeutic protocols can also be updated automatically using a local artificial intelligence algorithm, executed by a specific and dedicated processor. The parameterization of the local neural network can also be altered by the action of a top layer of artificial intelligence executed in the cloud. At this level, the global dynamics and efficacies, of all therapies, of all patients treated by the system, are constantly assessed and categorized.

important to reiterate that the invention fits partially into the group stimulation equipment electrical not transcutaneous invasive but moves away from any type of equipment electromedical available in 1 currently in the art, because it is a set of innovations that is configured in a whole new therapeutic technique called, in this order of privilege invention, the system Activation and Monitoring Neuromuscular Artificial, the acronym Samna. This new technique stands out, among other things, for its ability to automatically determine the appropriate therapy dosimetry for each group m uscular of each patient, respecting the variability intra and inter-patients.

The technology comprised by the invention can be USER SETTINGS to for recovery polytrauma individuals with lesions partial and / or complete spinal cord in clinics f isioterapia, or preventing acquired muscle weakness and polineuromiopatia critical illness in centers for intensive care or hospital beds ; in addition, it can be used in nursing homes for the elderly or similar applications. Another feature unique and innovative of the present invention, which distinguishes at all in the prior art, is in the structure of IoT, which enables the system to other novel features such as h omecare, b Usiness i ntelligence, m ineração of d ed (data mining), among many others.

### BACKGROUND OF THE INVENTION

Electrical stimulation has several applications, both for therapeutic and diagnostic purposes. Electrical stimulation can be applied to the central and peripheral nervous system, in addition to the musculotendinous system (Zaehle T1, Rach S, Herrmann CS. Transcranial alternating current stimulation enhances individual alpha activity in human EEG. PLoS One 2010; 5 (11): e13766. ; Routsi C, Gerovasili V, Vasileiadis I, et al. Electrical muscle stimulation prevents critical illness polyneuromyopathy : a randomized parallel intervention trial. Crit Care 2010 ; 14: R74).

The application technique can be invasive, using needle electrodes, or non-invasive, using transcutaneous electrodes (Lacomis D. Electrophysiology of neuromuscular disorders in critical illness. Muscle Nerve. 2013; 47 (3): 452-463.; Basu. I, Robertson MM, B Crocker et al linear Consistent and non-linear responses to invasive electrical brain stimulation and across primate species Individuals with implanted electrodes Brain. Stimul 2019; pii : S1935- 861X (19) 30086-5.; Li Q, Brus- Ramer M, Martin JH, McDonald JW. Electrical stimulation of the medullary pyramid promotes proliferation and differentiation of oligodendrocyte progenitor cells in the corticospinal tract of the adult rat. Neurosci Lett 2010; 479 (2): 128- 33).

Regarding the diagnostic application, electrical stimulation can detect changes in excitability and conduction in the nervous and muscular systems (Lacomis D. Electrophysiology of neuromuscular disorders in critical illness. Muscle Nerve. 2013; 47 (3): 452 -463).

For therapeutic purposes, neuromuscular electrical stimulation, also known in the art as NMES, or NeuroMuscular Electrical Stimulation, can be used to increase neuromuscular and osteotendinous trophism, increase muscle strength and increase blood flow (Maffiuletti NA, Minetto MA, Farina D, Bottinelli R. Electrical stimulation for neuromuscular testing and training: state-of-the art and unresolved issues. Eur J Appl Physiol 2011; 111: 2391-7.; Bieuzen F, Pournot H, Roulland R, Hausswirth C. Recovery after high-intensity intermittent exercise in elite soccer players using VEINOPLUS sport technology for blood flow stimulation J. Athl Train 2012; 47 (5): 498-506.; Hammond ER, Metcalf HM, McDonald JW, Sadowsky CL. Bone mass in individuals with chronic spinal cord injury: associations with activity-based therapy, neurologic and functional status, a retrospective study. Arch Phys Med Rehabil 2014; 95 (12): 2342-9).

The use of electric currents to improve the neuromuscular performance, by means of strength and mass, has been described in the literature for over 30 years (Kotz YM, Rodionov IM, Sitnikov BF, Tkhorevsky VI Vinogradova OL. On The Mechanism of an increase of muscle performance and of vasodilation during emotional stress in man. Pflugers Arch 1978 Mar 20; 373 (3): 211-8.; Freehafer AA, Peckham PH, Keith MW. New concepts on treatment of the upper limb in the tetraplegic. Surgical restoration and functional neuromuscular stimulation. Hand Clin. 1988; 4 (4): 563-74). In this context, several protocols are found with the most varied adjustments in the current parameters and in the most diverse functional disorders: both in animals and in humans. The studies are contrasting, presenting favorable results, but also indicating studies where electrical stimuli do not promote the expected neuromuscular performance (Poulsen JB, Møller K, Jensen CV, et al. Effect of transcutaneous electrical muscle stimulation on muscle volume in patients with septic shock. Crit Care Med 2011; 39 (3.): 456-61.; Gruther W Kainberger F Fialka -Moser V Paternostro-Sluga T Quittan M Spiss. C., et al Effects of neuromuscular electrical stimulation on muscle layer thickness of knee extensor muscles Patients in intensive care unit: a pilot study J. Med. Rehabil Med 2010; 42 (6):. 593-7).

It is known that the effectiveness of NMES to generate increased strength and, subsequently, neuromuscular hypertrophy is directly related to the intensity of the stimulus and the level of muscle contraction evoked, as well as to the volume of treatment applied. The stimulus intensity is directly correlated to the power of the equipment and its proper application depends on the applicator experience in making one fine tuning dess s parameters, to achieve shrinkage levels that actually promote tropism. In other words, the current literature shows that in therapies that apply stimuli with sufficient intensity to just keep the muscle contraction visible, there are no real gains for the patient's neuromuscular system. The contraction must be maintained at an intense enough level to have a real metabolic expenditure and below values that can cause injuries, there being a commitment relationship to be met. The therapeutic volume is determined by the number of times the stimuli are delivered over a predetermined time and it is desirable to be automatically controlled.

Although there is the aforementioned state of the art exploratory of the techniques related to the present invention, a significant number of studies report a relevant problem: some studies have failed to show significant gains in neuromuscular performance in patients with neuromuscular electrophysiological disorders.

This fact is due to the lack of equipment / technologies capable of delivering electrical stimuli to generate the intensity and volume of contraction necessary to improve neuromuscular performance and other systems. The authors Kern et al. (Kern H, Hofer C, Modlin M, et al. Denervated muscles in humans: limitations and problems of currently used functional electrical stimulation training protocols. Artif Organs. 2002; 26 (3): 216-8) were the first to suggest need for equipment with specific characteristics for use in certain populations. In line with this thought, Silva et al. (Silva PE, Babault N, Mazullo JB, et al. Safety and feasibility of a neuromuscular electrical stimulation chronaxie-based protocol in critical ill patients: A prospective observational study. J Crit Care. 2017; 37 : 141-8) demonstrated in their study the need to develop appropriate equipment for patients with neuromuscular electrophysiological disorders. The equipment available on the market, because they do not have enough channels, the ability to generate effective stimuli, to automate therapy, they do not have dynamic systems of security and dosimetry control for each muscle group independently, make the application of any type unfeasible. electrotherapy in this context.

In addition to the magnitude of the electrically evoked contraction to improve neuromuscular performance, the volume of stimuli balanced in time intervals makes all the difference to the success of therapy. Dow and colleagues (Dow DE, Cederna PS, Hassett CA, et al (2004) Number of contractions to maintain mass and force of a denervated rat muscle. Muscle Nerve 30: 77-86) tested the effect of NMES volume on muscles of animals with surgically denervated hind legs. In this study, the authors submitted guinea pigs to NMES sessions for 24 hours, for five weeks. Guinea pigs were allocated to different groups according to the number of electrically evoked contractions per day. An electric stimulator was implanted on the back of the guinea pigs and the contractions were divided in equal times throughout the 24 hours, for example: the group that received 200 contractions in 24 hours presented a contraction evoked every 7.2 minutes. In the study, muscle mass was maintained in groups that received between 200 to 300 contractions divided at equal intervals in 24 hours.

However, the same authors stated that this amount of contractions could vary between individuals and from one muscle group to another over the entire therapeutic period. At the moment, this type of treatment is not viable in humans, as there are no equipment capable of horizontally monitoring neuromuscular responsiveness and, from there, automatically reprogramming themselves to deliver a safe and effective treatment that must be continuously personalized. For this, permanent control of the applied current density and the level of evoked muscle contraction is necessary (Silva PE, Babault N, Mazullo JB, Oliveira TP, Lemos BL, Carvalho VO, et al. Safety and feasibility of a neuromuscular chronaxie-based electrical stimulation protocol in critical ill Patients: a prospective observational study J. Crit Care 2017; 37: 141-8.; Krenn M, Haller M Bijak. M, et al Safe electrical neuromuscular stimulator designed for the elderly. Artif Organs. 2011; 35 (3): 253-6).

The lack of continuous monitoring of neuromuscular responsiveness confers risks in the application of therapy, such as the occurrence of muscle contractures or injuries associated with an overdose, especially in cases of application in non-responsive or non-collaborative patients (Krenn M, Haller M, Bijak M, et al. Safe neuromuscular electrical stimulator designed for the elderly. Artif Organs. 2011; 35 (3): 253-6).

Regarding the diagnosis of neuromuscular electrophysiological disorders, several techniques have been used. The main ones are electroneuromyography with a needle electrode (invasive), the study of nerve conduction (non-invasive) and direct muscle stimulation, which can be invasive or non-invasive (Lacomis D. Electrophysiology of neuromuscular disorders in critical illness. Muscle Nerve. 2013; 47 (3): 452-463).

In addition to these, the TEDE (Test electrodiagnostic of stimulus) has been gaining popularity in recent years. From these tests it is possible to establish the diagnosis and the prognosis of electrophysiological neuromuscular disorders (Lacomis D. Electrophysiology of neuromuscular disorders in critical illness. Muscle Nerve 2013; 47 (3):. 452-463.; Fernandes LFRM Oliveira NML, Pelet DCS, et al. Stimulus electrodiagnosis and motor and functional evaluations during ulnar nerve recovery. Braz J Phys Ther 2016; 20 (2): 126-32).

Currently, equipment cannot perform all of these tests. For the realization of TEDE it is necessary to use specific devices. This is a test with good sensitivity when compared to the gold standard, which is electroneuromyography with a needle electrode, with the benefit of not being invasive and easy to apply (Paternostro-Sluga T, Schuhfried O, Vacariu G, Lang T, Fialka -Moser V. chronaxie and accommodation index in the diagnosis of muscle denervation. Am J Phys Med Rehabil 2002; 81: 253-60).

Despite the TEDE be a nonspecific marker where it is not possible to establish the cause of the injury, but only if there is injury, he has an advantage over the others : the basis of the results obtained in this test, it is possible to establish better metrics for NMES treatments (Silva PE, Babault N, Mazullo JB, Oliveira TP, Lemos BL, Carvalho VO, et al. Safety and feasibility of a neuromuscular electrical stimulation chronaxie-based protocol in critical ill patients: a prospective observational study. J Crit Care 2017 ; 37: 141-8).

TEDE is based on the diagnostic principle of the different responses evoked by the nerve and muscle with predetermined stimulation parameters (Paternostro-Sluga T, Schuhfried O, Vacariu G, Lang T, Fialka- Moser V. Chronaxie and accommodation index in the diagnosis of muscle denervation. Am J Phys Med Rehabil 2002; 81: 253 -60). To evoke muscle contraction, excitation of the presynaptic neuron is necessary. And it is q uatro the parameters evaluated in the stimulus electrodiagnostic test: rheobase, chronaxie, accommodation and lodging index.

The minimum stimulus-shaped rectangular pulse and infinite pulse width (1. 000 milliseconds) to reach the excitability threshold, that is, to evoke a visible minimal shrinkage, is called rheobase (Paternostro-Sluga T, Schuhfried O, Vacariu G, Lang T, Fialka- Moser V. Chronaxie and accommodation index in the diagnosis of muscle denervation. Am J Phys Med Rehabil 2002; 81: 253-60). Chronaxia, on the other hand, is the smallest rectangular pulse width necessary to evoke the minimum visible contraction, using as intensity twice the value of the reobase (Paternostro-Sluga T, Schuhfried O, Vacariu G, Lang T, Fialka- Moser V. Chronaxie and accommodation index in the diagnosis of muscle denervation. Am J Phys Med Rehabil 2002; 81: 253-60).

After denervation, the values of chronaxia become higher, since the evoked contractions are no longer acquired from the activation of the presynaptic nerve, but from the direct activation of muscle fibers, which require greater energy. In healthy muscles, their respective pre-synaptic nerve fibers trigger electrical stimulation, while in denervated muscles, it is the muscle fibers that react to the stimulus. The nerves have lower values of chronaxia than muscle fibers, therefore, innervated muscles have smaller chronaxes. The average chronaxia value of an innervated muscle varies from 60 to 200µs on average.

Accommodation is the property that the healthy muscle has of not responding, or of responding only with high intensities, to the pulses of exponential growth being measured as the lowest intensity necessary to produce a muscle contraction, evoked by an exponential pulse of infinite width (1st. 000 milliseconds) (Paternostro-Sluga T Schuhfried O Vacariu G, T Lang, Fialka -Moser V. chronaxie and accommodation index in the diagnosis of muscle denervation. Am J Phys Med Rehabil 2002; 81: 253 -60). The accommodation exposes the different responses of nerves and muscles to electrical pulses in exponential format.

In nerve fibers, an electrical pulse with an exponential shape, up to twice the value of the reobase, inactivates the sodium conductance before depolarization is reached, thus, no contraction is evoked. In the case of muscle fibers, sodium conductance is less altered by exponential pulses. In this way, it is possible to evoke muscle contractions with exponential pulses of intensity less than twice the reobase (Paternostro-Sluga T, Schuhfried O, Vacariu G, Lang T, Fialka- Moser V. Chronaxie and accommodation index in the diagnosis of muscle denervation. Am J Phys Med Rehabil 2002; 81: 253 -60). The accommodation index is the relationship between accommodation and reobase.

There is a direct relationship between human electrophysiological characteristics and the response to NMES. Thus, protocols with frequencies of 100 Hz and pulse widths of 1 have been suggested. 000 µs for healthy individuals, with the aim of evoking more torque. This strategy of NMES was called wide-pulse high-frequency (Neyroud D, Gonzalez M, Mueller S, et al. Neuromuscular adaptations to wide-pulse neuromuscular high-frequency electrical stimulation training. Eur J Appl Physiol 2019;. 119 (5) : 1105-16). However, it is a proposed protocol for i healthy ndividuals, since these values had normal to chronaxie. In patients with neuromuscular electrophysiological disorders, these values may be greater than 10,000 µs (Silva PE, Babault N, Mazullo JB, et al. Safety and feasibility of a neuromuscular electrical stimulation chronaxie-based protocol in critical ill patients: A prospective observational study. J Crit Care. 2016 ; 37: 141-148). For example, in the ICU, patients commonly develop polyneuromyopathy of critical illness, which leads to changes in neuromuscular excitability.

Thus, the use of NMES protocols with pulse widths based on chronaxia has been advocated for the treatment of these muscles, instead of pre-established values (Silva PE, Babault N, Mazullo JB, Oliveira TP, Lemos BL, Carvalho VO, et al. Safety and feasibility of a neuromuscular electrical stimulation chronaxie-based protocol in critical ill patients: a prospective observational study. J Crit Care 2017 ; 37: 141 -8).

The state of the art describes devices unable to provide safety and efficient and effective automations for the therapies described.

The equipment available on the market or the technologies claimed in patent documents were not designed to carry out these types of therapies with the level of safety and automation proposed in the present invention.

This happens because these devices do not have a series of innovations, which were integrated and implemented through the present invention with a focus on the aforementioned therapies, such as: (i) security system that determines automatically and in real time the current density adequate to protect the skin of patients in critical condition, even identifying whether the electrodes are out of use and / or poorly positioned; (ii) system of pre-programmed therapies and automatically readjusted according to the temporal needs of each muscle group; and (iii) specific processors connected to the internet to execute artificial intelligence algorithms, used both for horizontal monitoring of therapeutic parameters, and for defining the appropriate dosimetry for each muscle group, enabling the remote realization of therapeutic protocols in diverse applications, such as example, h omecare.

Furthermore, none of the devices available in the state of the art have technology capable of automatically evaluating and applying the appropriate dose to evoke an effective contraction, respecting the existing compromise relationship between stimuli with a intensity high enough to evoke vigorous contractions, necessary to achieve effective results, but not so high as to cause damage to the patients' muscular and / or epithelial structures. This was made possible by the present invention through the development of a system capable of identifying the precise level of muscle contraction and not only if the muscle presents muscle contraction or not.

The following are documents of the state of the art that comprise technologies related to the field of application of the present invention, but diametrically distinct.

The state of the art is described as a way of exemplifying its significant distance from the innovations presented by the present application for the privilege of invention.

The Brazilian patent document BR102017026510 describes a multichannel electrical muscle stimulation device to combat muscle weakness acquired in CTI, which has a biofeedback system implemented through an inertial sensor used to determine whether the muscle is contracted or not. The system uses the biofeedback sensor to automatically adjust the intensity of the stimuli when they stop contracting due to fatigue. In addition, the system has a hardware solution for activating different muscle groups simultaneously with biphasic waves. However, it differs from the present invention because the referid the document patent has no local or remote artificial intelligence algorithm to monitor horizontally neuromuscular condition of the patient. This prevents the automatic adjustment of therapeutic protocols, making it impossible to adapt to the different phases of therapy and requiring operator intervention.

In addition, the referred document does not have a system or resource that makes it possible to measure myography by electrical impedance, which allows structurally assessing the geometric changes that occur in the muscle at different intensities of contraction. In addition, said patent does not have a firmware algorithm capable of using bioimpedance measurements to assess neuromuscular responsiveness, as is done in the present invention. In addition, the technology described depends on an electromyographic sensor, inertial or similar, to assess the presence or absence of muscle contraction, while the present invention makes use of the muscle electrically activated electrodes to infer myographic activity through bioimpedance, without the need to fix any other type of device / electrode on the patient's skin.

Additionally, it should be noted that the present invention is not limited to informing whether or not there is contraction, but also informs the level of muscle activity according to the classification suggested by Segers and collaborators (Segers J, Hermans G, Bruyninckx F, et al. Feasibility of neuromuscular electrical stimulation in critically ill patients. J Crit Care 2014; 29: 1082-8). This feature allows the automatic determination of the most appropriate dosimetry, making the therapy more efficient and providing greater safety through protection against, for example, overdosing.

In addition, the document d and patent Brasileiro the BR102017026510 not have IoT feature, which makes it impossible t anto applications and remote updates, as sending data to the cloud that feeds a second level of artificial intelligence responsible for monitoring and control globally all systems connected as it is embodied in the present invention. Similarly, the device shown in the document of patent Brasileiro the BR102017026 not have cloud system for storage and data mining, as well as the artificial intelligence algorithm execution for global control of therapy for each patient and muscle group so independent. Finally, in the technology in question there is no artificial neuromuscular activation system based on current density, which is one of the unique characteristics of the present invention.

D this way, the device described in patent document cited is unable to identify the electrode wear level used or when they are incorrectly positioned, which ends by neglecting fundamental issues of security that allow the realization of long therapies duration. Given the above, it is clear that said patent comprises device and system distinguished are the technologies presented herein, it is not ing, of course, a Samna but, and solely a neuromuscular electrical stimulator multi with basic level automatism.

In addition, the US patent document US6324432 describes an electrical muscle stimulation device for sports applications, especially for passive training and re-education of atrophied muscle tissues. This device has sensors, of the electromyographic type and accelerometer, to measure the reactions of the stimulated muscle groups.

However, unlike mind the present invention, said device does not allow the measurement in real time of the muscular contraction level for automatic adjustment of the neuromuscular stimulation dosage most appropriate for a particular muscle group, and even enables control through the applied current density. Furthermore, it technology with limited number of channels, which are at most 2, and is completely absent u m safety system to prevent the application of noxious stimuli to the skin or stimuli to promote the emergence of damage due to d and overdosage. Finally, the referid the document has no artificial intelligence algorithms to automatically adjust the therapeutic protocols et am little resources of system of IoT, those present in the invention now required.

It is important to mention the US patent document US8565888, which presents a device for electrical stimulation of muscle tissues. It describes technology that aims to adapt the electrical stimulus based on the characteristics of the muscle response, such as fatigue. These control parameters are detected by means of sensors, such as accelerometers and extensometers. As described in the document, the device produces stimuli with a maximum of 400 microseconds of pulse width and 120 milliamps of amplitude of electric current in loads of a maximum of 1. 000 Ohm. In comparison with said equipment, the present invention provides electrical stimuli with a pulse width significantly greater than 400 microseconds, in addition to an intensity also greater than 250 milliamps in loads of up to 1. 000 Ohm, which allows the effective and efficient stimulation of denervated muscles and / or with changes in excitability.

In addition, the aforementioned document US8565888 does not have technology that allows the application of therapeutic protocols for continuous periods and exceeding 24 hours in duration, without the risk of the appearance of skin irritations or even injuries. Similarly, the technology described does not have a security system which prevents ça the application of noxious stimuli to the skin or to promote the emergence of injuries as a result d and overdose.

Finally, document US8565888 shows no artificial intelligence algorithms to automatically adjust the therapeutic protocols et am bit IoT features present in the invention herein claimed. In view of the above, it is clear that said patent does not include any of the innovative characteristics of the technology developed and described in the present invention.

In this tuning fork, equipment for muscle electrostimulation, composed of an array of electrodes and sensors, whose morphology is developed to minimize the need for accurate positioning of the electrodes is described in the US patent document US8285381.

It is said in that document that the device enables the prevention of burns by electrostimulation, as the sensors detect small increases in temperature that can generate damage to muscle tissues. However, it should be noted that the mere detection of temperature may not be sufficient to rule out the risk of injury to the patient's skin. Although the technology described in said document addresses temperature control, there is no resemblance to the present invention. The technology presented is not capable, unlike the required moment invention to generate stimuli with pulse widths of 400 microseconds and will not has sensors for measurement of electromechanical activity of the muscle-tendon joint, it would allow, as occurs in either the claimed technology, closed-loop control of stimulation parameters, when applied over long periods of therapy.

Additionally, the mentioned document does not include technology that allows electrostimulation programs, for continuous periods and exceeding 24 hours in duration, without the risk of the appearance of skin irritations or even injuries. The document also does not have a security system which prevents ça effectively and effective application of noxious stimuli to the skin or to promote the emergence of injuries as a result d and overdose. Finally, there is no mention of the artificial intelligence algorithms to automatically adjust the therapeutic protocols et am just the IoT features, such as the invention of this specification. In view of the above, it is clear that said document is not a system in the form as claimed in the present invention.

Finally, mention is made of the US patent document US20160051817, which describes equipment for muscle electrostimulation that proposes the generation of electrical stimuli controlled by pulses. Thus, the system it proposed seeks to generate the same level of muscle contraction that equipment today, however, making use of smaller amplitude current. For this, this system samples the stimulus applied to the patient and controls, via firmware, the amplitude of the applied voltage pulse. In addition to the stimulus intensity control mechanism of the referred document diverging from that present in the invention claimed here, the technology is not capable of generating electrical stimuli during long periods of electrostimulation and, like the others described, it does not have a security system that prevents the application. of noxious stimuli to the skin or to promote the emergence of injuries as a result d and overdose.

Just as the entire state of submitted art, the document above has no artificial intelligence algorithms to automatically adjust the therapeutic protocols et am just uses resources IoT of present and integrated in an innovative, effective and efficient in current invention.

Given the above, taking into consideration the representative examples of the prior art, it is clear that not exist one system activation and monitoring neuromuscular Artificial (Samna) intelligent and functional as the development in technology claimed by the present application of privilege of invention.

It is noteworthy that synergy promoted by integrating the developed system of tools IoT and the AI is not only innovative in, but adds to the state of the art technology is a significant advance in the field artificial neuromuscular stimulation and provides novel solutions to the existing problems in the state of the art, and aprimor will -1 it greatly.

### SUMMARY OF THE INVENTION

The present invention is characterized by a series of innovations which constitute in a novel therapeutic and diagnostic technique sometimes referred to as Activation and monitoring system Artificial Neuromuscular or Samna, based on artificial intelligence. SAMNA does not belong to the group of conventional medical electrical stimulation systems and equipment for treatment and diagnosis. This is because the present invention employs a number of innovations that enable, automatically, the precise diagnosis and promote effective treatment and customized for each type of muscle group in different patients.

SAMNA aims to continuously monitor the neuromuscular condition, in addition to treating and preventing vascular, osteotendinous and neuromuscular disorders from artificial and automated neuromuscular activation using cloud data processing involving b ig d ata and machine learning (machine learn). The present invention allows the diagnosis and treatment of neuromuscular disorders continuously, local or remote mind using technology IoT (Internet of Things). This is due to its unique hardware, firmware and software resources that allow the acquisition of neurophysiological parameters and the application of therapeutic parameters in an automatic, precise and safe way.

The Samna carries out its actions independently, personalized and coordinated, based on number s historic s data from the horizontal monitoring by Myography electrical impedance associated with automated stimulus electrodiagnostic test without the use sensors attached to the patient's body, using the electrotherapy electrodes themselves.

All of these features, in addition to ensuring safety through real-time control of the applied current density, assess the specific needs of each muscle group based on the neuromuscular response evoked during activation.

Thus, the system and technology developed ensures m viability of these features / functionality and implementation of effective and safe doses for treatment of functional changes associated with restricted mobility.

### BRIEF DESCRIPTION OF THE FIGURES

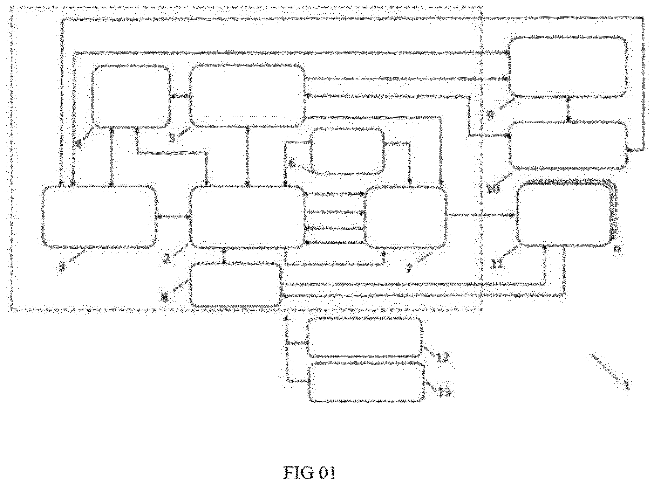

Since the present invention involves obtaining innovative results from the system developed in view of the state of the art, it is essential to demonstrate in images the constructive dispositions involved, which is done by means of the figures included in the present application patent. Thus, for a better understanding of the present invention, it was illustrated in Figures 1 to 7, not being restricted as to the dimensions, dispositions, colors and formats presented.
Figure 1 shows the diagram of the artificial neuromuscular activation system (1), highlighting the following elements: central processing unit (2), communication unit (3), artificial intelligence processor (4), human - machine (5), emergency button (6), power unit (7), electrical impedance acquisition system (8), user device (9), web server (10), electrodes (11), power supply (12) and battery (13). The battery is optional.

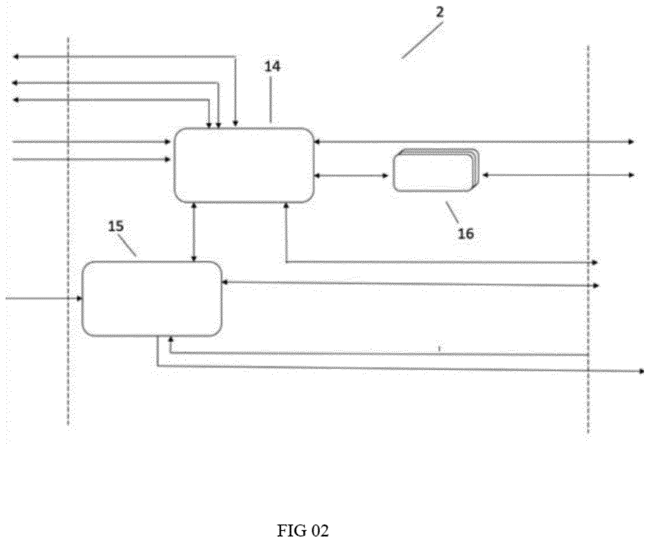

Figure 2 shows the diagram of the central processing unit (2), highlighting the following elements: control unit (14), security unit (15) and digital analog converter (16).

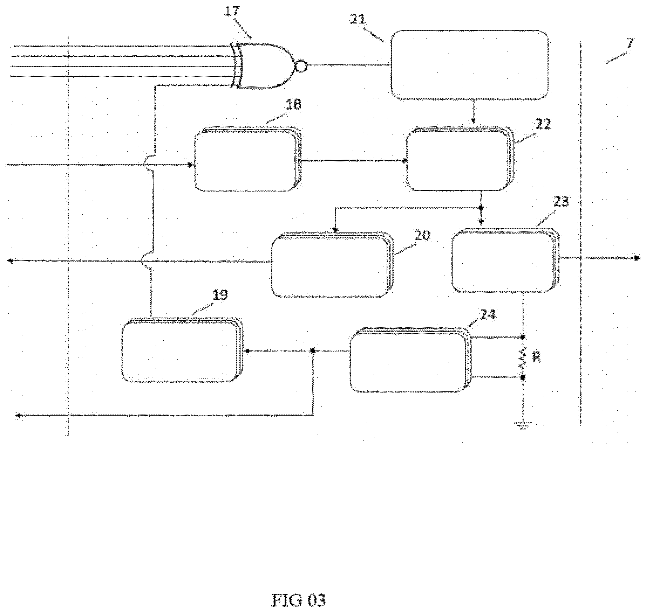

Figure 3 shows the diagram of the power unit (7), with the following elements: global safety switch (17), current voltage converter (18), comparator circuit (19), voltage meter circuit (20), high voltage regulator (21), high voltage generator (22), switching unit (23) and current meter circuit (24).

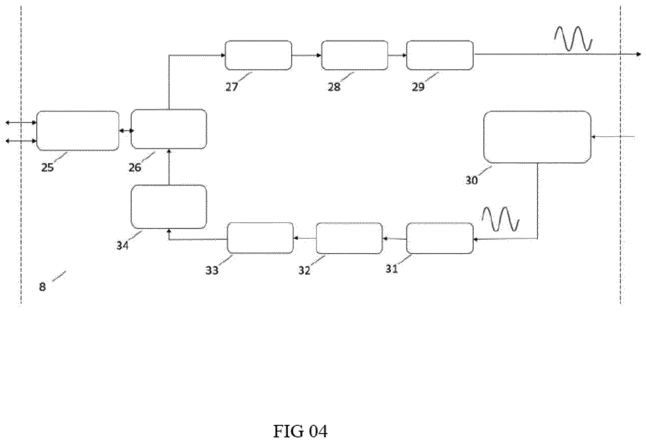

Figure 4 shows the electrical impedance myography acquisition system (8), highlighting the impedance processor (26), the direct digital synthesis - DDS (27), analog to digital converter (28), programmable gain circuit output (29), artifact filter (30), programmable input gain circuit (31), anti aliasing filter (32), digital analog converter (33), discrete Fourier transform processor (dft) (34) and device communication (25).

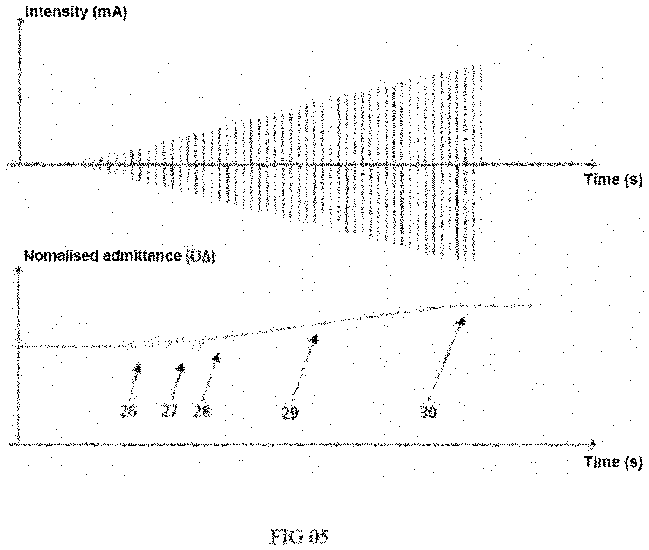

Figure 5 shows two graphs: graph A represents a stimulation ramp, wherein each trace vertical performed is a stimulus and graph B represents the response of admittance measured at the time a muscle stimulated by graph A. ramp We highlight the points of contraction level 1 (26), contraction level 2 (27), contraction level 3 (28), contraction level 4 (29) and contraction level 5 (30).

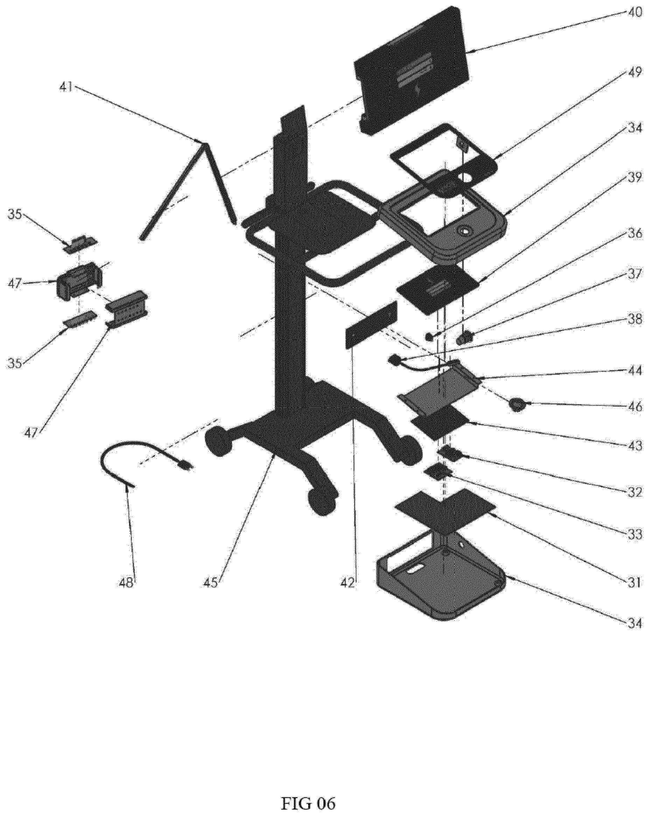

Figure 6 shows the mechanical parts of the artificial neuromuscular activation system (1) in exploded view, highlighting: Main board ha rdware (31), power source (32), drive D C D C (33) cabinet (34), connectors (35), power button (36), emergency button (37), ethernet cable (38), touch screen display (39), monitor (40), swing arm (41), parent level parameters (42), the controller board display (43), displa plate holder y (44) c arrinho structure with wheels and support structure (45), the knob button type (46), case d the connection electrodes (47), power cable (48) and front seal label (49).

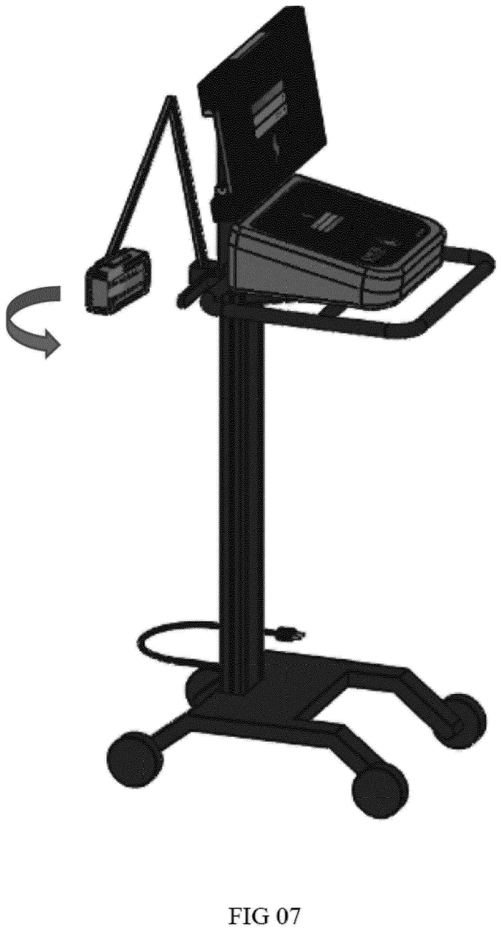

The Figure 7 shows an overview of Samna, with highlight to its moving parts.

### DETAILED DESCRIPTION OF THE INVENTION

The Samna (1) is a summarized system in a device electromedical mobile composed of one structural trolley and the supporting structure (45) case (34), where there main board M ardware (31), source (32), DC to DC converter (33). An articulated arm (41) takes the case of the connection of the electrodes (47) with the connectors for the electrostimulation cables (35) close to the patient..

The present invention has a man - machine interface (5) composed of a touch screen display (39) and its display controller board (43) located in the cabinet (34), monitor (40), on-off button (36) and ethernet connection (38) located in the parameter panel (42), emergency button (37) and a knob type button (46) for navigation.

For better ergonomics, it is possible to adjust the height position d and the electrode connector box (47) through the articulated arm, such as shown in Figure 7.

The feeding Sanma (1) is performed by the C power abo (48) that connects not the source aliment the dog (32) automatic dual voltage. There is the possibility to add a battery (optional item) for the system power.

The artificial neuromuscular activation system (SAMNA) (1) reads the myographic activity through the same electrode pairs (11) used in the electrostimulation performed in therapies and diagnoses. Des s shape, the system eliminates the need for external sensors. The acquisition of myography is made by the electrical impedance acquisition system (8), a technique known as Electrical Impedance Myography (MIE), at an acquisition rate of up to 1000 samples per second.

The reading of the MIE is commanded by the central processing unit (2) by means of commands sent to the impedance processor (26), using the communication device (25) of the electrical impedance myography acquisition system (8).

At the beginning of a myography acquisition, the impedance processor (26) sends the command to the direct digital synthesis - DDS (27) which, together with the analog digital converter (28), generates a sine wave with adjustable frequency of 10Hz to 100MHz, with peak to peak amplitude of 2V. This signal passes through the programmable output gain circuit (29) and is sent to the skin by means of a pair of electrodes (11), in which one of them applies the current to the skin and the other performs the collection, submitting this signal to a artifact filter (30), implemented in hardware, which has the dual function : removal of artifact to stimulus and protection of the signal acquisition system. This filter is especially important when the muscle is being electrically stimulated by the power unit (7), as it removes the high voltage artifact generated by neuromuscular electrostimulation. The filtered signal then passes through the signal conditioning system composed of a programmable input gain circuit (31) and anti aliasing filter (32). The conditioned signal is acquired through the digital analog converter (33). The discrete Fourier transform processor - dft (34) accumulates 1. 024 values and applies the formula, where: x (f) is the signal strength at point f; x (n) is the digital analog converter output; sen (n) and cos (n) are values from the DDS core at point f.

The acquired impedance values are then sent to the impedance processor (26), which calculates the impedance module and phase. After this step, the obtained values are sent to the central processing unit (2), through the communication device (25), which determines the level of muscle contraction and, later, these values are sent to the artificial intelligence processor (4).

To determine the level of contraction, the central processing unit (2) assesses the average of a history series of impedance values taken before the stimulus, the impedance values measured during the stimulus and inherent variation from the baseline of the measured impedances.

The Samna (1) is capable of performing the stimulus electrodiagnostic test (TEDE) using procedures that determine the r eobase, the chronaxie and the accommodation form manual or automatic, and thus evaluate neuromuscular condition of each group m us cul ar to be evaluated.

In automatic mode, the pulses are controlled by the central processing unit (2) through the power unit (7) and muscle contraction is identified by changing the muscle geometry measured by the electrical impedance acquisition system (8).

In the manual mode, the setting parameters of the pulses is performed by the operator and the values generated are the central processing unit (2) by means of the power unit (7) and subsequently the muscle contraction is visually identified by operator.

In both cases, the assessment of neuromuscular condition by muscle is performed by the artificial intelligence processor (4) using the data received from chronaxia, reobase and accommodation of the series of impedance values measured during the electrodiagnostic test of stimulus (TEDE) and also using the patient data informed by the operator in the human - machine interface (5).

SAMNA (1) guarantees safety in electrostimulation throughout the TEDE by assessing the density of applied current and checking the position and quality of the electrodes.

In the automatic TEDE mode, sequential procedures are performed for each muscle, with thirteen steps in all.

In step (a), the electrical impedance acquisition system (8) performs 500 impedance measurements without electrical stimulation, that is, with the muscle relaxed, consisting of the measurements in the set of measures A.

In step (b), the central processing unit (2) initiates, through the power unit (7), the generation of a pulse width 1. 000ms, initial intensity of 1mA and resting time of 2. 000ms. Simultaneously, the Myography acquisition system for electrical impedance (8) 500 performs impedance measurements, consisting this s the set of measures B.

Further, in step (c), the central processing unit (2) calculates the standard deviation of each set of measurements obtained in the previous steps (A and B) and discards the values differing according to the following criterion: values greater than or equal to twice the standard deviation (SD) value of the respective set of measures. After the rejection of the outlier points, the means, medians and standard deviations of the two groups are recalculated and these values are sent to the fisher's quadratic classifier to make the decision as to whether or not there was muscle contraction. The determination of the classifier boundaries is carried out using data obtained during the process of determining the levels of muscle contraction, which will be detailed below.

In step (d), if the decision generated in step (c) was "no contraction", the intensity is increased according to the binary search algorithm with an initial value of 10mA and the steps are repeated from (a) to (d). If the decision is for "identification of contraction" the value of the reobase is defined as the intensity of the last stimulation performed, which is registered and sent to the human - machine interface (5) and the process is followed by step (e).

In sequ is INSTANCE, there is step (e), wherein the electrical impedance acquisition system (8) carries 500 impedance measurements without electrical stimulation, ie with relaxed muscles, thereby generating the set of measures group C.

In step (f), the central processing unit (2) through the power unit (7) starts generating a pulse with an intensity of twice the value found for the reobase and initial pulse width of 20 microseconds, with a resting time of 2. 000 milliseconds. Simultaneously, the electrical impedance acquisition system (8) performs 500 impedance measurements, generating the set of measurements in group D.

In step (g), the central processing unit (2) calculates the standard deviation of the points acquired in the set of measurements in groups s C and D and rejects points that differ more than twice the standard deviation of the respective group. After the rejection of the points, the means, medians and standard deviations of both groups are recalculated and these values are sent to the fisher's quadratic classifier to make the decision as to whether or not there was muscle contraction.

Thus, n step (h), c f the decision is "there was no contraction", the pulse width is increased according to the algorithm binary search with an initial value of 50 microseconds and repeats m up the steps from (e) to (h). If the decision is for contraction, the chronax value is defined as the pulse width of the last stimulation performed and this value is recorded and sent to the HMI (5) and the process is followed by step (i).

Proceed to step (i), where the myography acquisition system by electrical impedance acquisition system (8) performs 500 impedance measurements without electrical stimulation, that is, with the muscle relaxed, obtaining the group E data set.

No step (j), the central processing unit (2) by means of the power unit (7) initiates the generation of an exponential stimulus strength twice the value found rheobase and duration 1. 000 microseconds. Simultaneously the electrical impedance acquisition system (8) performs 500 impedance measurements, obtaining the group F data set.

In step (k), the central processing unit (2) calculates the standard deviation of the acquired points are group are E and F, discarding the points that differ by more than 2 standard deviations. After the rejection of the points, the means, medians and standard deviations of both groups are recalculated and these values are sent to the fisher's quadratic classifier to make the decision as to whether or not there was muscle contraction. If the decision is due to no contraction, the intensity is increased according to the binary search algorithm with an initial value of 10mA and the steps from (i) to (k) are repeated. If the decision is for contraction, the accommodation value is defined as the intensity of the last stimulation performed and this value is registered and sent to the human machine interface (5) and the procedure is followed. The From this point, the central processing unit (2) then calculates the map index using the formula. This value is registered and sent via the human - machine interface (5).

Finally, in step (1), the central processing unit (2) then sends the values obtained for chronaxia, reobase, accommodation, the accommodation index, the series of impedance values measured during the Electro Test d Stimulus diagnosis (TEDE) and patient data informed by the operator through the human - machine interface (5) for an artificial intelligence processor (4).

Then, in step (m), a TEDE report and the assessment of the neuromuscular condition for each muscle is made available to the operator, on the human - machine interface (5) and on the web server (10).

The present invention is able to measure and maintain the level of muscle contraction during the therapies performed. For this, before starting a therapy, SAMNA (1) automatically identifies the stimulation parameters capable of generating muscle contractions at levels from 1 to 5, for each muscle. For this task, the system generates, through the central processing unit (2) and the power system (7), a sequence of stimuli with increasing intensity, in a linear ramp. The electrical impedance acquisition system (8) will perform data acquisitions throughout this stimulation process. From the analysis of the acquired data, SAMNA (1) correlates the bioimpedance values with each of the contraction levels, following the reference shown in Figure 5. Between levels 3 and 5 it is still possible to identify a series of sub-levels if required.

Level 1 of contraction is related to the sets of bioimpedance measures that have a standard deviation twice that of that observed in the set of measures taken while the muscle was at rest (26). However, when the standard deviation of the measurements taken is equal to 3 times the standard deviation at rest, the central processing unit (2) determines that the intensity value is sufficient for level 2 contraction (27).

A stretch of linear impedance growth then begins. Level 3 is given by the intensity value that starts the growth line shown in Figure 5 (28). Level 4 is set to the middle of the straight growth (29) and level 5 as the intensity value on the line of inflection shown in F igure 5 constant value for the impedance (30).

After 10 intensity increments without any impedance variation, contraction level 5 is detected and the ramp is interrupted.

SAMNA (1) has a security system for real-time control of the applied current density. This functionality prevents the appearance of burns and skin lesions, even when the system is applied in therapies or diagnostics of critically ill patients.

For this, before applying each of the electrical current stimuli, the central processing unit (2) uses the data received by the electrical impedance acquisition system (8) and the type / dimensions of the electrodes used (informed by the operator), correlating them with the contact area and then calculating the electric current density using the formula:, where d is the current density; i is the pulse intensity; 1 is the pulse width, and f is the frequency of the stimulus.

The safe current density value (d) to be applied to the skin must be less than or equal to 3.5 mA / cm². In this way, the system will continuously monitor the stimulus and, if the value of the density needed to evoke a certain level of contraction reaches this limit, a message is displayed on the human - machine interface (5) requesting the replacement of the electrodes.

During any diagnostic or therapy procedure, the central processing unit (2) evaluates the average of the impedance values acquired between the stimulations (with the muscle at rest) sent by the electrical impedance acquisition system (8). If the central processing unit (2) detects a growth rate greater than 10%, the system considers that there was a problem with the electrode, such as loss of the electrolyte or detachment. Thus, the central processing unit (2) temporarily interrupts the therapy or diagnosis by generating a warning on the human - machine interface (5) to change or reposition the electrodes (11). After changing / repositioning the electrodes (11), the operator resumes therapy by pressing the "play" button on the human machine interface (5).

As this additional security feature, SAMNA (1) is subject to yet another layer of protection that will only allow the system to operate if all of its main subsystems are operating correctly. This is done by means of its global safety switch (17) which is responsible for activating and deactivating the high voltage regulator (21) of the power unit (7), man - machine interface (5), comparator circuit of the power unit power (19), electrical impedance acquisition system (8) and emergency button (6). In this way, if any of the main subsystems detects a failure or if the operator presses the emergency button, the power part of SAMNA (1) is immediately disabled, rendering it unable to generate stimuli.

The local artificial intelligence system, through dedicated processor, is constantly monitoring the time until the onset of muscle fatigue, monitoring the responsiveness of muscles to stimulation, the level of contraction, r eobase the chronaxie and accommodation, allowing automatic decision-making throughout the therapeutic period.

Data from electrodiagnostics and therapies performed, as well as patient information registered through the HMI or the web server, are sent to the artificial intelligence (AI) processor (4).

Through its trained artificial neural network, the AI processor (4) generates a therapy suggestion that is shown to the operator through the human - machine interface (5) containing pulse information such as intensity, pulse width, frequency, time of ascent, time of stimulation, time of descent and time of rest; in addition to treatment information, such as number of stimuli in therapy, type of therapy and number of indicated therapies per day. The operator has the possibility to make any adjustments he deems necessary.

With each therapy and diagnosis performed, the electrical impedance acquisition system (8) performs new collections that are analyzed by the AI processor (4). As a product of this procedure, the system offers an adaptation in the proposal of the next therapy.

SAMNA (1) is capable of performing automatic firmware and software updates over the internet that allows the insertion of new therapeutic protocols and new functionalities to the system.

can be updated individually or collectively: the application of the interface man - machine (5), the firmware control unit (14), the firmware security unit (15), firmware communication unit (3), software the artificial intelligence processor (4), and the firmware of the electrical impedance acquisition system (8).

The data generated in the therapies and diagnostics, as well as the data collected by the electrical impedance acquisition system (8) and the suggestions for therapies generated by the artificial intelligence processor (4) are sent periodically to the web server (10).

The data on the server is accessed by two systems: Vision and Oracle. The first is a webapp that allows the operator to view and add new data to the web. The second is performed within a supervised learning classification artificial intelligence algorithm. Supervision is carried out by a team of physiotherapists and doctors specialized in electrostimulation.

From time to time, the parameters of the new network trained on the web are Unsent the via internet to all devices to replace the existing neural network used locally in equipment, making the increasingly robust device for carrying out the diagnosis and treatment.

## Claims

1. - System for enabling and monitoring based on artificial neuromuscular artificial intelligence comprising one structural cart with wheels and support structure (45) case (34) provided with adapter hardware (31), power source (32), converter DC DC (33), an articulated arm (41) takes the case of connection of electrodes (47) with the connectors of cables electrical stimulation (35), a man-machine interface (5) to display touch screen (39) and a display controller board (43) allocated n the cabinet (34), the monitor (40) and provided with articulated arm (41), for moving case of connection of electrodes (47), on-off button (36) and connection ethernet (38) located in the parameter panel (42), emergency button (37) and knob type button (46) for navigation.

2. - SYSTEM, according to claim 1, **characterized by** comprising mechanisms of continuous and simultaneous stimulation of osteotendinous and vascular tissues, said mechanisms promoting electrical activation of peripheral or central nervous tissue, and muscle tissue from different dermatomes in a manner automatic.

3. - SYSTEM according to claim 1, **characterized in that** is provided with engine control d current density power applied (in mA / centímetro²) maintaining said density equal to or less than 3, 5 m A / cm².

4. - SYSTEM according to claim 1, **characterized by** an artificial intelligence algorithm built, with an automatic calibration and therapeutic protocols performed by means of said allocated server algorithm in cloud structure.

5. - PROCESS, according to claim 1, **characterized by** comprising the activation of the central processing unit (2) by means of commands sent to the impedance processor (26) and delivered by the communication device (25) to the myography system by electrical impedance (8) for reading the myographic activity using pairs of electrodes (11) at the acquisition rate of up to 1,000 samples per second, with the beginning of each myography acquisition being processed by the substeps of:
• sending by command impedance processor (26) to direct digital synthesis - DDS (27) ;
• joint generation from direct digital synthesis - DDS (27) and the digital analog converter (28) of a sign wave with adjustable frequency from 10Hz to 100MHz ;
• passage of the signal generated by the programmable output gain circuit (29) and sent to the patient's skin through a pair of electrodes (11), in which one of them applies the current to the skin and the other performs signal collection and vice -version;
• submitting the collected signal to an artifact filter (30);
• removing the device from high voltage generated by the neuromuscular electrostimulation, obtaining the filtered signal;
• passage of a voltage signal from a sample of the applied stimulus, through a programmable input gain circuit (31) and anti aliasing filter (32), obtaining the conditioned signal through the subsequent passage in a digital analog converter (33);
• impedance calculation by dedicated processing unit, impedance processor (26);
• and by means of a communication device (25) of the values obtained to the central processing unit (2) determining the level of muscle contraction, the said determination being made through the evaluation of the historical series of impedance values taken before the are e module, the impedance values measured during the stimulation and the inherent variation of line b ase of measured impedances; and
• sending the muscle contraction level data to the artificial intelligence processor (4).

6. - PROCESS according to claim 1 **characterized by** the automatic mode comprising pulse control by the central processing unit (2) and by the power unit (7), the detection of the minimum muscle contraction being identified through the impedance acquisition system electrical (8).

7. - PROCESS according to claim 1 **characterized by** the manual adjustment of the parameters by the operator and the identification of the muscular contraction being visual.

8. - PROCESS according to claim 1 **characterized by** the evaluation of the neuromuscular condition of the muscle to be performed by means of an artificial intelligence processor (4) from the data of the parameters reobase, chonaxia, and accommodation of the series of impedance values measured during the electrodiagnostic test of stimulus and / or from data reported by the operator on the human-machine interface (5).

9. - PROCESS according to claim 1, comprising sequential procedures for each muscle in the steps of:
a) performance by the electrical impedance acquisition system (8) of 500 impedance measurements without electrical stimulation, consisting of the measurements obtained in the set of measures A;
b) generation of a 1,000ms wide pulse, initial intensity of 1mA and resting time of 2,000ms from the central processing unit (2) and the power unit (7), and simultaneous performance of 500 impedance measurements by means of the electrical impedance myography acquisition system (8), consisting of the measurements obtained in the set of measures B;
c) calculation in the central processing unit (2) of the standard deviation of each of the sets of measures obtained in the previous steps, in A and B, with values greater than or equal to twice the value of the standard deviation of the respective set of measures discarded and subsequent recalculation of means, medians and standard deviations of the two groups, the values obtained being sent to the fisher's quadratic classifier, determining the occurrence or not of muscle contraction;
d) if muscle contraction is not seen in (c), the pulse intensity is increased according to the binary search algorithm with an initial value of 10mA and the steps from (a) to (d) are repeated; if muscle contraction is verified, the rheobase value is defined as the intensity of the last stimulation performed, which is registered and sent to the human-machine inte rface (5) and follows step (e);
e) conducting 500 impedance measurements using the electrical impedance acquisition system (8) without electrical stimulation, thus obtaining the group C measurements ;
f) generation of a pulse with an intensity of twice the value found for the reobase and initial pulse width of 20 microseconds, with a resting time of 2,000 milliseconds by the central processing unit (2) and power unit (7) and simultaneous realization of 500 impedance measurements by electrical impedance acquisition system (8), obtaining the set of group D measures ;
g) calculation of the standard deviation of the points acquired in the set of measurements of groups C and D by the central processing unit (2), with the points that differ more than twice the standard deviation of the respective group discarded, subsequently being recalculated. averages, medians and standard deviations of both groups and the values obtained sent to the fisher's quadratic classifier, determining the occurrence or not of muscle contraction;
h) if muscle contraction is not observed in (g), the pulse width is increased according to the binary search algorithm with an initial value of 50 microseconds and the steps from (e) to (h) are repeated ; if muscle contraction is verified, the chronax value is defined as the pulse width of the last stimulation performed, which is registered and sent to the human-machine interface (5) and follows step (i);
i) performs tion of 500 impedance measurements by Myography acquisition system through the electrical impedance acquisition system (8) without electrical stimulation, obtaining the data set of the group E ;
j) generation of an exponential stimulus with intensity of twice the found value of reobase and duration of 1,000 microseconds from the central processing unit (2), through the power unit (7), being carried out simultaneously by the acquisition system electrical impedance (8) d 500 measurements and impedance, yielding the data set of F group ;
k) calculation of the standard deviation of the points acquired in groups E and F in the central processing unit (2), rejecting the points that differ more than 2 standard deviations, being subsequently calculated new means, medians and standard deviations of both groups and the values sent to the fisher's quadratic classifier, determining the occurrence or not of muscle contraction, and if no contraction is verified, the intensity is increased according to the binary search algorithm with an initial value of 10mA and the steps of (i) to (k) and checked the contraction, the accommodation value is defined as being the intensity of the last stimulation performed, which is recorded and sent to the interface man - machine (5), calculating n the central unit processing (2) the accommodation index, the respective value being registered and sent to the human-machine interface (5) ;
l) sending the central processing unit (2) d values obtained from chronaxie, the rheobase, the map, the map index, the series of measured impedance values for the electrodiagnostic test stimulus and patient data entered by the operator via the human-machine interface (5) for an artificial intelligence processor (4) ; and
m) disponibilization to the operator in the man-machine interface (5) and the web server (10) of a report electrostimulation test and d evaluation of neuromuscular condition for each muscle.

10. - SYSTEM, according to claim 1, **characterized by** having a mechanism for safety against burns and / or skin irritations by means of the control, in real time, of the applied current density, said mechanism being activated by the central control unit. processing (2), generating information visible on the human-machine interface about overdose and about the exchange or repositioning of the electrodes (11).

11. - PROCESS according to claim 1, **characterized by** comprising a mechanism for automatic identification of the presence of critical structural changes and the decoupling of the electrodes, and through said mechanism adjustments are made to the intensity and/or pulse width and/or frequency of the stimulus and/or pre-established protocol by the operator and/or disabled the electrostimulation channel(s) that are outside the safety parameters.
